(19) Europäisches Patentamt
European Patent Office
Office européen des brevets

(11) **EP 4 607 471 A1**

(12) **EUROPEAN PATENT APPLICATION**

(43) Date of publication:
**27.08.2025  Bulletin 2025/35**

(21) Application number: **24159273.2**

(22) Date of filing: **23.02.2024**

(51) International Patent Classification (IPC):
**G06V 10/25** (2022.01)   **G06V 10/82** (2022.01)
**A61B 1/00** (2006.01)   **A61B 5/107** (2006.01)
**G06T 7/00** (2017.01)

(52) Cooperative Patent Classification (CPC):
**G06V 10/25; A61B 1/000096; A61B 5/1076;
G06T 7/62; G06V 10/82; G06T 2207/10068;**
G06T 2207/30096; G06V 2201/032

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Medintech Inc.**
**Seoul 03100 (KR)**

(72) Inventors:
• **LEE, Chi Won**
  **Namyangju-si, Gyeonggi-do (KR)**
• **KIM, Myung Joon**
  **Gwacheon-si, Gyeonggi-do (KR)**
• **KIM, Tae Hyeon**
  **Seoul (KR)**
• **CHO, Seok Ho**
  **Seoul (KR)**
• **KIM, Dong Hyeok**
  **Seoul (KR)**

(74) Representative: **Gualeni, Nadia**
**Jacobacci & Partners S.p.A.**
**Piazza della Vittoria, 11**
**25122 Brescia (IT)**

(54) **ENDOSCOPIC DEVICE AND METHOD FOR OBTAINING LESION SIZE INFORMATION**

(57)   Provided are an endoscopic device for identifying a lesion based on a pre-trained model and determining size information on the identified lesion, and a method of obtaining size information on a lesion.

The method of obtaining size information on a lesion includes obtaining an image inside a body from an image sensor, identifying at least one lesion from the image based on a pre-trained model, and determining size information on the lesion.

FIG. 1

**Description**

[Technical Field]

The disclosure relates to an endoscopic device and a method of obtaining size information on a lesion, and more particularly, to an endoscopic device for identifying a lesion and determining size information on the identified lesion based on a pre-trained model, and a method of obtaining size information on a lesion.

[Background Art]

An endoscopic device is a medical instrument for inserting a scope into a human body to observe various organs and perform a treatment or procedure as needed. A user using an endoscopic device usually simultaneously manipulates a scope and performs a medical operation of checking for abnormalities inside a human body. In such a situation where the user's concentration is dispersed, the user may miss a lesion that requires observation or treatment, depending on the user's skill level or fatigue.

In addition, when a lesion is discovered during investigation using an endoscopic device, the response methods depend on the size of the discovered lesion, and thus, it is necessary to measure the size of the lesion. In the related art, the size of a lesion is measured with the naked eye, which results in low accuracy.

The above-mentioned background art is technical information possessed by the inventor for the derivation of the disclosure or acquired during the derivation of the disclosure, and cannot be considered as a known technique disclosed to the general public prior to the filing of the disclosure.

[Disclosure of Invention]

[Technical Problem]

Provided are an endoscopic device for identifying a lesion based on a pre-trained model and determining size information on the identified lesion, and a method of obtaining size information on a lesion.

However, the above objective is an example, and the objectives of the disclosure are not limited thereto. Other objectives than the above may be clearly understood by those of skill in the art from the present specification and the accompanying drawings.

[Solution to Problem]

According to an aspect of the disclosure, a method of obtaining size information on a lesion includes obtaining an image inside a body from an image sensor, identifying at least one lesion from the image based on a pre-trained model, and determining size information on the lesion.

In the present embodiment, the method may further include calculating a first distance, and the determining of the size information on the lesion may include determining the size information on the lesion based on the first distance.

In the present embodiment, the calculating of the first distance may include calculating the first distance based on brightness information on the lesion expressed in the image.

In the present embodiment, the calculating of the first distance may include obtaining a first image when a tip portion is at a first angle, and a second image when the tip portion is at a second angle, and measuring a difference between the first angle and the second angle, and a visual disparity between the first image and the second image.

In the present embodiment, the identifying of the lesion may include determining lesion size information on the image, which indicates size information on the lesion on the image.

In the present embodiment, the lesion size information on the image may include a bounding box displayed to surround the lesion.

In the present embodiment, the method may further include controlling the tip portion to track the lesion.

In the present embodiment, the angle of view of the image sensor may include a first area, and the controlling of the tip portion may include controlling the tip portion such that the lesion is located within the first area.

In the present embodiment, the controlling of the tip portion may include calculating a first value.

According to another aspect of the disclosure, an endoscopic device includes a tip portion including an image sensor, and a control unit configured to obtain an image inside a body from the image sensor, identify at least one lesion from the image based on a pre-trained model, and determine size information on the lesion.

In the present embodiment, the control unit may be further configured to calculate a first distance, and determine the size information on the lesion based on the first distance.

In the present embodiment, the control unit may be further configured to calculate the first distance based on brightness information on the lesion expressed in the image.

In the present embodiment, the control unit may be further configured to obtain a first image when the tip portion is at a first angle, and a second image when the tip portion is at a second angle, and measure a difference between the first angle and the second angle, and a visual disparity between the first image and the second image.

In the present embodiment, the control unit may be further configured to determine lesion size information on the image, which indicates size information on the lesion on the image.

In the present embodiment, the lesion size information on the image may include a bounding box displayed to surround the lesion.

In the present embodiment, the control unit may be further configured to control the tip portion to track the lesion.

In the present embodiment, the angle of view of the image sensor may include a first area, and the control unit may be further configured to control the tip portion such that the lesion is located within the first area.

In the present embodiment, the control unit may be further configured to calculate a first value.

Other aspects, features, and advantages than those described above will become clear from the following detailed description, claims, and drawings for carrying out the disclosure.

[Advantageous Effects of Invention]

An endoscopic device and a method of obtaining size information on a lesion according to an embodiment may detect a lesion inside a body without being affected by a user's skill level and fatigue, by using a pre-trained model to identify the lesion.

According to the endoscopic device and the method of obtaining size information on a lesion according to an embodiment, by automatically determining size information on an identified lesion, the endoscope device may obtain size information on the lesion accurately and consistently without deviation due to the user's experience and skill level, and based on this, it is possible to select a correct coping method for the lesion, and thus improve the quality of treatment.

The effects of the disclosure are not limited to those described above, and other effects not described herein may be clearly understood by one of skill in the art from the present specification and the accompanying drawings.

[Brief Description of Drawings]

FIG. 1 is a diagram illustrating an endoscopic device according to an embodiment.
FIG. 2 is a diagram illustrating an example in which a pre-trained model identifies a lesion from images inside a body, according to an embodiment.
FIG. 3 is a diagram illustrating an image output on a display unit when a lesion is identified.
FIG. 4 is a diagram illustrating a first distance inside a body.
FIG. 5 is a diagram illustrating the concept of obtaining size information on a lesion based on lesion size information on an image.
FIG. 6 is a diagram illustrating an image output on a display unit when a lesion is located in a first area.
FIG. 7 is a diagram illustrating a method of calculating a first value based on a position difference on images according to a difference in angle of a tip portion.
FIG. 8 is a flowchart of a method of obtaining size information on a lesion, according to an embodiment.
FIG. 9 is a flowchart showing sub-operations of calculating a first distance, according to an embodiment.
FIG. 10 is a flowchart of a method of obtaining size information on a lesion, according to another embodiment.
FIG. 11 is a flowchart showing sub-operations of controlling a tip portion, according to an embodiment.

[Mode for Invention]

Terms used herein are for describing particular embodiments and are not intended to limit the scope of other embodiments. The singular expression may also include the plural meaning as long as it is not inconsistent with the context. All the terms used herein, including technical and scientific terms, may have the same meanings as those generally understood by those of skill in the art. The terms as defined in a general dictionary may be interpreted as the same meanings as the contextual meanings of a related technology, and are not interpreted as ideal or excessively formal meanings unless defined clearly in the disclosure. In some cases, even the terms defined in the disclosure should not be interpreted to exclude the embodiments of the disclosure.

Hereinafter, various embodiments will be described in detail with reference to the accompanying drawings for those of skill in the art to be able to implement the embodiments without any difficulty. The disclosure may, however, be embodied in many different forms and is not limited to the embodiments set forth herein. When it is determined that, in describing embodiments disclosed herein, detailed description on function or configuration of related known technologies may unnecessarily confuse the gist of the disclosure, the detailed description will be omitted. The same or similar components will be assigned the same reference numeral and redundant descriptions thereof will be omitted.

The term "... unit" or "...er (or)" as used herein refers to a component configured to perform a particular function that is performed by software or hardware such as a field-programmable gate array (FPGA) or an application-specific integrated circuit (ASIC). However, the term "... unit" or "...er (or)" is not limited to being performed by software or hardware. The term "... unit" or "...er (or)" may exist in the form of data stored in an addressable storage medium, or may be implemented by instructions such that one or more processors execute a particular function.

The software may include a computer program, code, instructions, or a combination of one or more thereof, and may configure the processor to operate as desired or may independently or collectively instruct the processor. Software and/or data may be embodied permanently or temporarily in any type of machine, component, physical or virtual equipment, computer storage medium or device, or in a propagated signal wave capable of providing instructions or data to or being interpreted by the processor. The software may be distributed on networked computer systems and stored or executed in a distributed manner. The software and data may be stored in one or more computer-readable recording media. The software may be read into a main memory from another computer-readable medium, such as a data storage device, or from another device via a communication interface. Software instructions stored in the main memory may cause a processor to perform processes or operations that will be described in detail below. Alternatively, hardwired circuitry may be used instead of or in combination with software instructions to execute processes consistent with the principles of the disclosure. Thus, embodiments consistent with the principles of the disclosure are not limited to any particular combination of hardware circuitry and software.

Terms used herein are merely used to describe a particular embodiment, and are not intended to limit the disclosure. Singular forms are intended to include plural forms as well, unless the context clearly indicates otherwise. As used herein, terms such as "comprises," "includes," or "has" specify the presence of stated features, numbers, stages, operations, components, parts, or a combination thereof, but do not preclude the presence or addition of one or more other features, numbers, stages, operations, components, parts, or a combination thereof. Terms such as "first" or "second" may be used to describe various elements, but the elements should not be limited by the terms. These terms are used only to distinguish one component from another.

The term 'learning model' as used herein may encompass any type of algorithm or methodology used to learn or understand a particular pattern or structure from data. That is, the term 'learning model' may encompass not only machine learning models such as regression models, decision trees, random forests, support vector machines, K-nearest neighbors, Naive Bayes, or clustering algorithms, but also deep learning models such as neural networks, convolutional neural networks, recurrent neural networks, transformer-based neural networks, generative adversarial networks (GANs), or autoencoders. The term 'learning model' may refer to a set of trained parameters or weights that are used to predict or classify an output for a particular input, and such a model may be trained by performing methods such as supervised learning, unsupervised learning, semi-supervised learning, or reinforcement learning. In addition, it may include not only a single model but also various learning methods and structures, such as ensemble models, multimodal models, and models through transfer learning. Such learning models may be pre-trained on a computer device separate from the computer device that predicts an output for an input, and used by another computer device.

A learning model according to an embodiment may include models for object detection and position estimation.

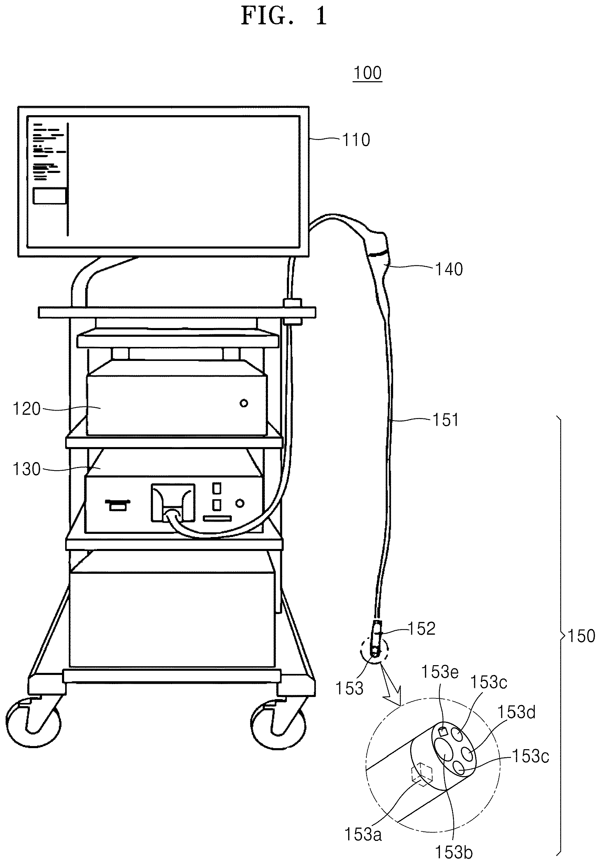

Hereinafter, an endoscopic device and a method of obtaining size information on a lesion according to an embodiment will be described with reference to FIGS. 1 to 11.

FIG. 1 is a diagram illustrating an endoscopic device 100 according to an embodiment.

The endoscopic device 100 is a medical instrument for inserting a scope into a human body to observe various organs and perform a treatment or procedure as needed. Referring to FIG. 1, the endoscopic device 100 may include a display unit 110, a control unit 120, a driving unit 130, a manipulation unit 140, and a scope 150.

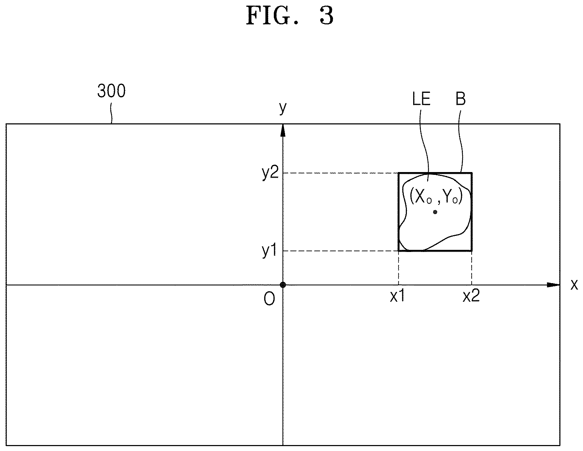

The display unit 110 may output an image. In other words, the display unit 110 may output an internal body image obtained from an image sensor 153a to be described below. The output internal body image may include x-axis coordinates and y-axis coordinates (see FIG. 3).

The display unit 110 may include a display module capable of outputting visualized information or implementing a touch screen, such as a liquid-crystal display (LCD), a thin-film transistor-LCD (TFT-LCD), an organic light-emitting diode (OLED) display, a flexible display, or a three-dimensional (3D) display.

The display unit 110 may output lesion size information S on the image, which will be described below.

The control unit 120 may control the overall operation of the endoscopic device 100. The control unit 120 may include all types of components capable of processing data. In an embodiment, the control unit 120 may include a hardware-embedded data processing device having a physically structured circuitry to perform functions represented by code or instructions included in a program. The hardware-embedded data processing device may include a processing device such as a microprocessor, a central processing unit (CPU), a processor core, a multiprocessor, an application-specific integrated circuit (ASIC), or a field-programmable gate array (FPGA).

The control unit 120 may determine size information R of an identified lesion. The control unit 120 will be described in detail below.

The driving unit 130 may provide power necessary for the scope 150, which will be described below, to be inserted into a body or to move inside the body. For example, the driving unit 130 may include a plurality of motors connected to wires within the scope 150, and a tension control unit configured to adjust the tension of the wires.

The manipulation unit 140 may input a command of a user. The manipulation unit 140 may include a plurality of buttons that provide various functions to control the angle of a tip portion 153, which will be described below, or to perform various procedures inside the body.

The scope 150 may be directly inserted into the body. In detail, the scope 150 may include an insertion unit 151, a bending portion 152, and the tip portion 153.

The insertion unit 151 may serve to insert the tip portion 153, which will be described below, to an arbitrary position inside the body that is subject to observation and treatment. The insertion unit 151 may be connected to one end of the manipulation unit 140.

The bending portion 152 may be connected to one end of the insertion unit 151. The bending portion 152 may serve to change the angle of the tip portion 153, which will be described below. The bending portion 152 may be flexibly bent. As the bending portion 152 is bent, the angle of the tip portion 153 may be changed. The bending portion 152 may be connected to the driving unit 130 to receive a force necessary to change the angle of the tip portion 153. The degree or direction of bending of the bending portion 152 may be determined by the driving unit 130.

The tip portion 153 may be connected to one end of the bending portion 152. The tip portion 153 may photograph the inside of the body, and perform a treatment or a procedure as needed. The tip portion 153 may include the image sensor 153a, a lens 153b, a light 153c, a working channel 153d, and an air/water channel 153e.

The image sensor 153a may serve to obtain an image inside the body. The image inside the body may include a video image consisting of a plurality of consecutive frames. The image inside the body may be output through the display unit 110.

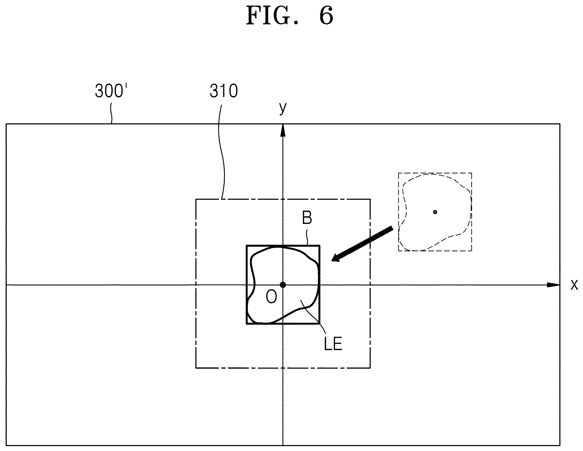

The angle of view of the image sensor 153a may include a first area. The first area may include the center of the angle of view. In an image 300' inside the body, a portion 310 corresponding to the first area may include the center of the image 300' (see FIG. 6). Thus, when a target is located in the first area, the target may be located close to the center of the image output through the display unit 110.

In an embodiment, the image sensor 153a may include a plurality of image sensors 153a.

The lens 153b may serve as a passage through which light reflected inside the body enters the image sensor 153a. The light 153c may emit light toward the inside of the body such that an image inside the body may be obtained by using the image sensor 153a. The number of lights 153c is not particularly limited. A tool for treating and processing a lesion LE may be inserted into the working channel 153d. Air or washing water may be supplied through the air/water channel 153e.

Hereinafter, the control unit 120 will be described in detail.

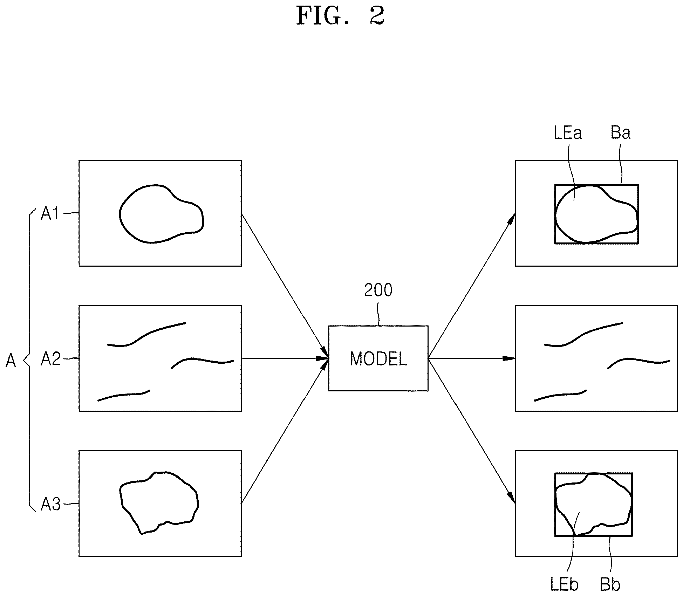

FIG. 2 is a diagram illustrating an example in which a pre-trained model 200 identifies the lesion LE from images A inside a body, according to an embodiment. FIG. 3 is a diagram illustrating the image 300 output on the display unit 110 when the lesion LE is identified.

The control unit 120 may include a processor configured to perform various calculations or operations to be described below. The processor may read a computer program to perform data processing for machine learning. The processor may perform computational processes such as processing input data for machine learning, extracting features for machine learning, or calculating errors based on backpropagation. The processor for performing such data processing may include a central processing unit (CPU), a general-purpose graphics processing unit (GPGPU), a tensor processing unit (TPU), an ASIC, an FPGA, or the like. However, this is only an example, and the type of the processor may be configured in various ways that may be understood by those of skill in the art based on the description.

The control unit 120 may obtain an image inside the body from the image sensor 153a, identify at least one lesion LE from the image based on the pre-trained model 200, and determine size information R of the lesion.

The control unit 120 may train the model in advance. The model may be trained to receive an image inside a body, identify a lesion LE from the image inside the body, and determine lesion size information S on the image. The model may receive images inside a body as training data. In addition, the model may receive label data corresponding to each image inside the body, with the size of a lesion indicated as a bounding box.

The model may include a network structure such as a fully convolutional network (FCN), a conditional adversarial network (CAN), a recurrent neural network (RNN), or a matching cost-CNN (MC-CNN).

Referring to FIG. 2, the control unit 120 may identify the lesion LE from the images A based on the pre-trained model 200. The control unit 120 may input, into the pre-trained model 200, the images A inside the body obtained from the image sensor 153a. The pre-trained model 200 having received the input of the images A inside the body may identify the lesion LE and determine and output the lesion size information S on the image. The lesion size information S on the image may be information indicating the size of the lesion LE on the image output to the display unit 110. In an embodiment, as illustrated in FIG. 2, the lesion size information S on the image may include a bounding box B displayed to surround the lesion LE.

For example, as illustrated in FIG. 2, when an image A1 inside the body is input to the pre-trained model 200, the pre-trained model 200 may identify a lesion LEa included in the image A1 inside the body, and output a bounding box Ba surrounding the lesion LEa. When an image A2 inside the body is input to the pre-trained model 200, the image A2 inside the body does not have a lesion, and thus, the pre-trained model 200 does not output a bounding box. When an image A3 inside the body is input to the pre-trained model 200, the pre-trained model 200 may identify a lesion LEb included in the image A3 inside the body, and output a bounding box Bb surrounding the lesion LEb.

In an embodiment, the lesion size information S on the image may include coordinate information on the image for four line segments of the bounding box B. That is, the pre-trained model 200 may determine the coordinate information on the image for the four line segments of the bounding box B.

For example, referring to FIG. 3, the pre-trained model 200 may calculate, as the lesion size information S on the image, an x-coordinate x1 of the left line segment of the bounding box B, an x-coordinate x2 of the right line segment of the bounding box B, a y-coordinate y1 of the lower line segment of the bounding box B, and a y-coordinate y2 of the upper line segment of the bounding box B. A horizontal size xw of the bounding box B is a value obtained by subtracting the x-coordinate x1 of the left line segment of the bounding box B from the x-coordinate x2 of the right line segment of the bounding box B (xw = x2 - x1). A vertical size yh of the bounding box B is a value obtained by subtracting the y-coordinate y1 of the lower line segment of the bounding box B from the y-coordinate y2 of the upper line segment of the bounding box B (yh = y2 - y1). The lesion size information S on the image may include the horizontal size xw of the bounding box B, and the vertical size yh of the bounding box B.

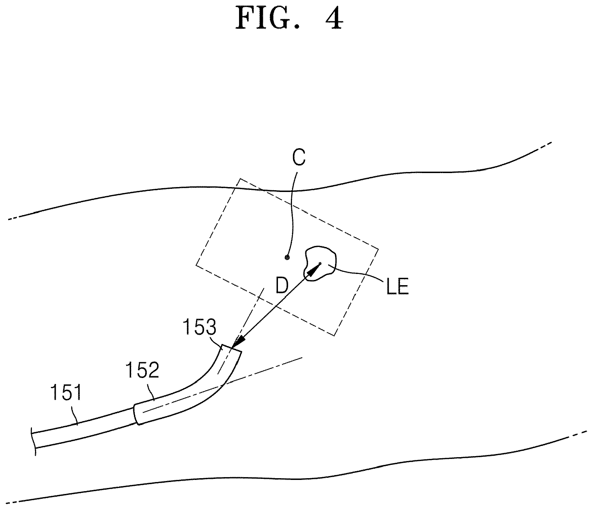

FIG. 4 is a diagram illustrating a first distance D inside a body.

The control unit 120 may calculate the first distance D. The first distance D may be a value necessary to determine the size information R of the lesion. In detail, the first distance D may serve as a medium connecting numerical information on the image to numerical information inside the actual body. The first distance D may include the distance between the lesion LE and the tip portion 153. In more detail, the first distance D may include the distance between the lesion LE and the lens 153b.

In an embodiment, the control unit 120 may calculate the first distance based on brightness information on the lesion LE expressed in the image. The distance from the tip portion 153 to an arbitrary target inside the body, and corresponding brightness data on the image in which the arbitrary target inside the body is reflected by the light 153c may be pre-stored in the control unit 120. Thus, by applying the brightness of the lesion LE on the image to the data, the distance from the tip portion 153 to the lesion LE, that is, the first distance D, may be calculated.

In an embodiment, the first distance D may be calculated by using an average. In detail, the first distance D may be calculated by averaging distances obtained by applying brightnesses at a plurality of points within the lesion LE, to the data.

In another embodiment, the control unit 120 may obtain a first image i1 when the tip portion 153 is at a first angle a, and a second image i2 when the tip portion 153 is at a second angle b, and measure a difference $\Delta\theta$ between the first angle a and the second angle b, and a visual disparity $\Delta L$ between the first image i1 and the second image i2.

The first angle a and the second angle b are two different arbitrary angles for calculating the first distance D, and may refer to angles between the tip portion 153 and the insertion unit 151 formed by the bending portion 152. Meanwhile, as the angle of the tip portion 153 changes, the position of the image sensor 153a may change, and as the position of the image sensor 153a changes, images may appear as if the position of an arbitrary point W changes. The visual disparity may refer to the degree to which the images appear as if the position of the arbitrary point W changes as the position of the image sensor 153a changes. In detail, the control unit 120 may calculate the first distance D by using [Equation 1] below.

[Equation 1]

$$D = \frac{R\Delta\theta \times f}{\Delta L}$$

In [Equation 1], $\Delta\theta$ denotes the difference between the first angle a and the second angle b, and $\Delta L$ denotes the visual disparity between the first image i1 and the second image i2. R denotes the radius of rotation at which the bending portion 152 is bent, and thus, $R\Delta\theta$ denotes the displacement of the image sensor 153a. f denotes the focal length of the lens 153b.

In another embodiment, the control unit 120 may calculate the first distance D by using a stereo method based on a plurality of image sensors 153a.

In another embodiment, the control unit 120 may emit, as structured light, light of a particular pattern, such as stripes or grids, toward the lesion LE, and calculate the first distance D by measuring the distortion of the pattern of the light returned from the lesion LE to the image sensor 153a.

In another embodiment, the control unit 120 may calculate the first distance D by measuring the travel time of light reflected from the lesion LE after emitting light to the lesion LE.

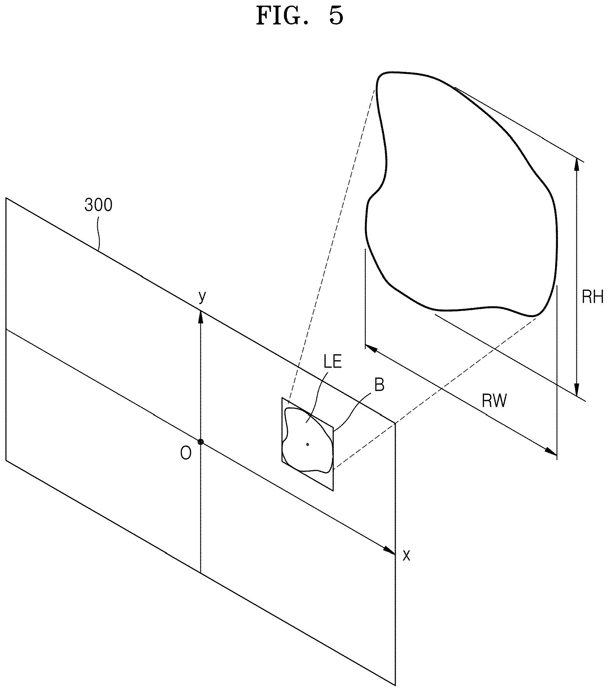

FIG. 5 is a diagram illustrating the concept of obtaining size information R of a lesion based on lesion size information S on an image.

The control unit 120 may determine the lesion size information R based on the first distance D. The control unit 120 may determine the size information based on the lesion size information S on the image and the first distance D.

In an embodiment, the control unit 120 may determine the size information R of the lesion by using a regression model and a proportional value of a size that the size of one pixel on the image actually corresponds to at a point away from the tip portion 153 by the first distance D. Referring to FIGS. 3 and 5, the size information R of the lesion may be obtained by multiplying the proportional value by the number of pixels corresponding to the size information on the image of the bounding box B surrounding the lesion LE. As a specific example, an actual horizontal size RW of the lesion may be calculated by multiplying the horizontal size xw of the bounding box B by the proportional value. An actual vertical size RH of the lesion may be calculated by multiplying the vertical size yh of the bounding box B by the proportional value.

In another embodiment, the control unit 120 may obtain the size information R of the lesion by using the first distance D and the focal length f of the lens 153b. The focal length f of the lens 153b, the sizes xw and yh of the bounding box B on the image, the first distance D, and the actual sizes RW and RH of the lesion may be expressed by an proportional expression as shown in [Equation 2] below.

[Equation 2]

$$xw\, f = RW : D$$

$$yh\, f = RH : D$$

Thus, the actual sizes RW and RH of the lesion included in the size information R of the lesion may be calculated by using [Equation 3] below, which is a modification of [Equation 2].

[Equation 3]

$$RW = \frac{(xw) \times D}{f}$$

$$RH = \frac{(yh) \times D}{f}$$

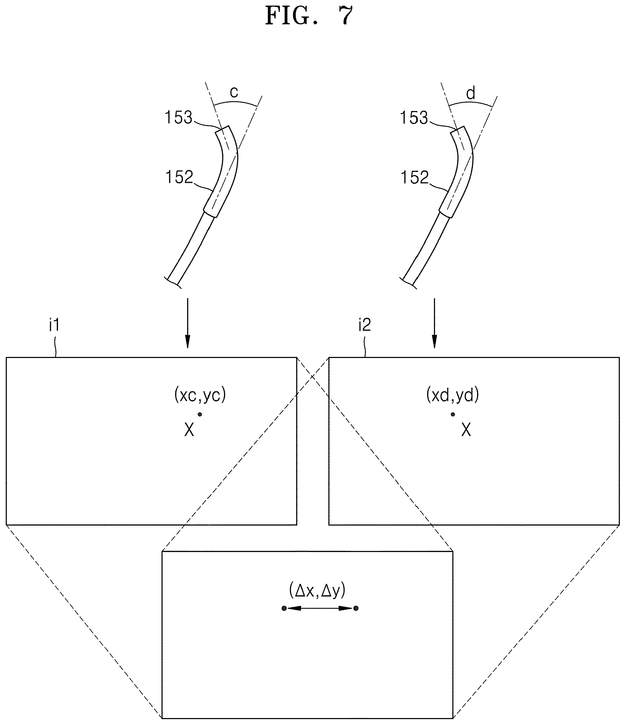

FIG. 6 is a diagram illustrating the image 300' output on the display unit 110 when the lesion LE is located in the first area. FIG. 7 is a diagram illustrating in detail a method of calculating a first value K based on a position difference on images according to a difference in angle of the tip portion 153.

The control unit 120 may control the tip portion 153 to track the lesion LE. In detail, as illustrated in FIG. 6, the control unit 120 may control the tip portion 153 such that the lesion LE is located within the first area. That is, as the tip portion 153 is controlled such that the identified lesion LE is located within the first area, the identified lesion LE may be located close to the center of the image 300' output through the display unit 110. As a result, the identified lesion LE may be continuously output on the display unit 110.

In an embodiment, the coordinates of the center of the bounding box B may be calculated from the coordinate information on the image for the four line segments of the bounding box B. Referring to FIG. 3, coordinates (xo, yo) of the center of the bounding box B of the lesion LE identified by the pre-trained model 200 in the image 300 inside the body output to the display unit 110 may be calculated as in [Equation 4] below from the coordinate information on the image for the four line segments of the bounding box B. The coordinates (xo, yo) of the center of the bounding box B may be referred to as the coordinates of the center of the identified lesion LE.

[Equation 4]

$$(x_o = \frac{x1 + x2}{2},\ y_o = \frac{y1 + y2}{2})$$

The control unit 120 may calculate the first value K, calculate a total movement angle TMA of the tip portion based on the coordinates (xo, yo) of the center of the lesion on the image and the first value K, calculate a target angle ag for the current time by considering a movement angular velocity as and a control period cp of the tip portion 153, and move the tip portion 153 by the target angle ag for the current time.

The control unit 120 may calculate the first value K. The first value K is the ratio of the difference in position on the image to the change in angle of the tip portion 153. The first value K may be a value necessary to calculate the total movement angle TMA of the tip portion 153.

In an embodiment, referring to FIG. 7, the control unit 120 may obtain a third image i3 when the tip portion 153 is at a third angle c, and a fourth image i4 when the tip portion 153 is at a fourth angle d, and measure a difference $\Delta\delta$ between the third angle c and the fourth angle d, and a position difference $\Delta X$ between the third image i3 and the fourth image i4.

The third angle c and the fourth angle d are two different arbitrary angles for calculating the first value K, and may refer to angles between the tip portion 153 and the insertion unit 151 formed by the bending portion 152. Meanwhile, the position difference $\Delta X$ between the third image i3 and the fourth image i4 may include a difference in coordinates on images for an arbitrary point X inside the body. That is, it may include the difference between the coordinates of the arbitrary point X on the third image i3 and the coordinates of the arbitrary point X on the fourth image i4.

Referring to FIG. 7, the control unit 120 may obtain the third image i3 with the image sensor 153a when the tip portion 153 is caused to be at the third angle c by manipulating the bending portion 152. Here, the coordinates of the arbitrary point X inside the body on the image may be (xc, yc). The method of designating the arbitrary point X is not particularly limited. In an embodiment, the arbitrary point X may be the position of a blood vessel having a particular shape inside the body found on the image.

Again, the control unit 120 may obtain the fourth image i4 with the image sensor 153a when the tip portion 153 is caused to be at the fourth angle d by manipulating the bending portion 152. Here, the coordinates of the arbitrary point X inside the body on the image may be (xd, yd).

The coordinate difference between the third image i3 and the fourth image i4 may be expressed as $(\Delta x, \Delta y)$, which is the difference between the coordinates of the arbitrary point X on the images. Here, $\Delta x = xd - xc$, and $\Delta y = yd - yc$. By using this, the position difference $\Delta X$ between the third image i3 and the fourth image i4 is expressed as [Equation 5] below.

[Equation 5]

$$\Delta X = \sqrt{(\Delta x)^2 + (\Delta y)^2}$$

By applying the difference $\Delta\delta$ between the third angle c and the fourth angle d and the position difference $\Delta X$ between the third image i3 and the fourth image i4 to [Equation 6] below, the first value K may be calculated.

[Equation 6]

$$K = \frac{\Delta X}{\Delta\delta}$$

In an embodiment, calculating the first value (S241) may use the first image i1 and the second image i2 obtained in calculating the first distance (S130). In detail, as the difference $\Delta\delta$ between the third angle c and the fourth angle d, the difference $\Delta\theta$ between the first angle a and the second angle b may be used. In addition, as the position difference $\Delta X$, the difference between coordinates (xa, ya) of the arbitrary point W on the first image i1 and coordinates (xb, yb) of the arbitrary point W on the second image i2 may be used.

The control unit 120 may calculate the total movement angle TMA of the tip portion based on the coordinates (xo, yo) of the lesion on the image, and the first value K. The total movement angle TMA refers to a total angle that the tip portion 153 needs to move to move the center coordinates (xo, yo) of the lesion LE to the center coordinates (0, 0) on the image. A length Q from the center of the lesion LE to the center of the image is as shown in [Equation 7] below.

[Equation 7]

$$Q = \sqrt{(x_0)^2 + (y_0)^2}$$

The total movement angle TMA may be calculated by applying the length Q from the center of the lesion LE to the center of the image, and the calculated first value K to [Equation 8] below.

[Equation 8]

$$(\mathrm{TMA}) = \frac{Q}{K}$$

The control unit 120 may calculate the target angle ag for the current time by considering the movement angular velocity as and the control period cp of the tip portion 153. The movement angular velocity as and the control period cp of the tip portion 153 may be set in advance.

In an embodiment, the target angle ag for the current time may be calculated through a polynomial trajectory. As a specific example, the tip portion 153 may be set to move by the total movement angle TMA at a constant velocity. The preset movement angular velocity as of the tip portion 153 may be 30°/s, and the preset control period cp may be 2 ms. In this case, the target angle ag for the current time may be 30°/s × 2ms = 0.2 °. However, this is only an example for description and the disclosure is not limited thereto.

In another embodiment, the target angle ag for the current time may be calculated through a Bezier curve trajectory. That is, the method of calculating the target angle ag for the current time is not limited to the polynomial trajectory. In other words, the target angle ag for the current time may be calculated through various trajectories that may occur to one of skill in the art.

The control unit 120 may move the tip portion 153 by the target angle ag for the current time. The control unit 120 may calculate a force required to move the tip portion 153 by the target angle ag for the current time, by considering the dynamic characteristics of the bending portion 152. The control unit 120 may deliver information about the calculated force to the driving unit 130 to move the tip portion 153 by the target angle ag for the current time.

As illustrated in FIG. 6, the control unit 120 may repeatedly perform the process of identifying the lesion, calculating the first distance D, and controlling the tip portion 153, until the lesion LE is located in the first area. Accordingly, the tip portion 153 may track the lesion LE, and the lesion LE may be continuously output on the display unit 110.

As the tip portion 153 is controlled such that the identified lesion LE is located within the first area, the lesion LE is located close to a point C at which the center of the field of view of the image sensor 153a is located, and thus, the convenience and accuracy of calculating the first distance D may be improved. Accordingly, the reliability of the size information R of the lesion may be improved. In addition, even a low-skilled user is able to track and observe the lesion LE without difficulty, and the quality of treatment may be improved as the user is able to focus on observation, treatment, and procedures of the identified lesion LE without having to pay attention to manipulation of the scope.

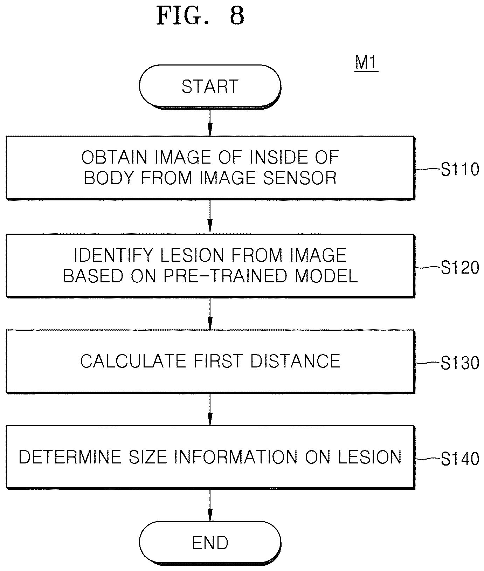

FIG. 8 is a flowchart of a method M1 of obtaining size information on a lesion, according to an embodiment.

The method M1 of obtaining size information on a lesion is a method of identifying a lesion LE in an image inside a body obtained from the endoscopic device 100, and obtaining size information on the identified lesion LE.

Referring to FIG. 8, the method M1 of obtaining size information on a lesion may include obtaining an image inside the body from the image sensor 153a (S110), identifying at least one lesion LE from the image based on the pre-trained model 200 (S120), calculating a first distance D (S130), and determining size information R of the lesion (S140).

The endoscopic device 100 may obtain an image A inside the body from the image sensor 153a (S110). By inserting the scope 150 into the body, an image inside the body may be obtained through the image sensor 153a provided at the tip portion 153. The image inside the body may include a video image consisting of a plurality of consecutive frames. The image inside the body may be output through the display unit 110.

Referring to FIG. 2, the endoscopic device 100 may identify the lesion LE from the image A based on the pre-trained model 200 (S120). The endoscopic device 100 may input the image A inside the body obtained from the image sensor 153a, into the pre-trained model 200. The pre-trained model 200 having received the input of the images A inside the body may identify the lesion LE and determine and output lesion size information S on the image. The lesion size information S on the image may be information indicating the size of the lesion LE on the image output to the display unit 110. In an embodiment, as illustrated in FIG. 2, the lesion size information S on the image may include the bounding box B displayed to surround the lesion LE.

For example, as illustrated in FIG. 2, when the image A1 inside the body is input to the pre-trained model 200, the pre-trained model 200 may identify the lesion LEa included in the image A1 inside the body, and output the bounding box Ba surrounding the lesion LEa. When the image A2 inside the body is input to the pre-trained model 200, the image A2 inside the body does not have a lesion, and thus, the pre-trained model 200 does not output a bounding box. When the image A3 inside the body is input to the pre-trained model 200, the pre-trained model 200 may identify the lesion LEb included in the image A3 inside the body, and output the bounding box Bb surrounding the lesion LEb.

The model may be trained to receive an image inside a body, identify a lesion LE from the image inside the body, and determine lesion size information S on the image. The model may receive images inside a body as training data. In addition, the model may receive label data corresponding to each image inside the body, with the position of a lesion indicated as a bounding box.

The model may include a network structure such as an FCN, a CAN, an RNN, or an MC-CNN.

In an embodiment, the lesion size information S on the image may include coordinate information on the image for four line segments of the bounding box B. That is, the pre-trained model 200 may determine the coordinate information on the image for the four line segments of the bounding box B.

For example, referring to FIG. 3, the pre-trained model 200 may calculate, as the lesion size information S on the image, the x-coordinate x1 of the left line segment of the bounding box B, the x-coordinate x2 of the right line segment of the bounding box B, the y-coordinate y1 of the lower line segment of the bounding box B, and the y-coordinate y2 of the upper line segment of the bounding box B. The horizontal size xw of the bounding box B is a value obtained by subtracting the x-coordinate x1 of the left line segment of the bounding box B from the x-coordinate x2 of the right line segment of the bounding box B (xw = x2 - x1). The vertical size yh of the bounding box B is a value obtained by subtracting the y-coordinate y1 of the lower line segment of the bounding box B from the y-coordinate y2 of the upper line segment of the bounding box B (yh = y2 - y1). The lesion size information S on the image may include the horizontal size xw of the bounding box B, and the vertical size yh of the bounding box B.

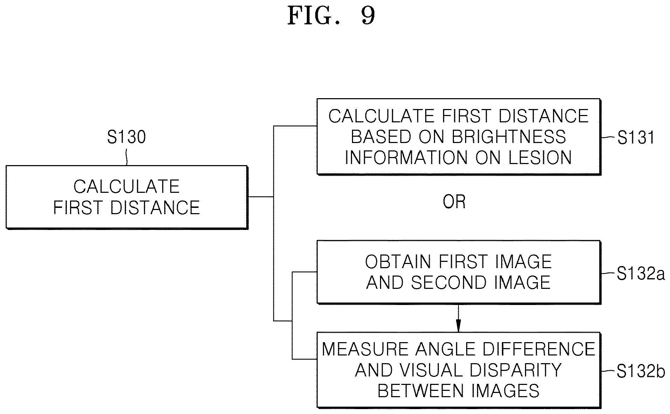

FIG. 9 is a flowchart showing sub-operations of the calculating of the first distance (S130), according to an embodiment.

The endoscopic device 100 may calculate the first distance D (S130). The first distance D may be a value necessary to determine the size information R of the lesion. In detail, the first distance D may serve as a medium connecting numerical information on the image to numerical information inside the actual body. The first distance D may include the distance between the lesion LE and the tip portion 153. In more detail, the first distance D may include the distance between the lesion LE and the lens 153b.

In an embodiment, referring to FIG. 9, the calculating of the first distance (S130) may include calculating the first distance based on brightness information on the lesion LE expressed in the image (S131). The distance from the tip portion 153 to an arbitrary target inside the body, and corresponding brightness data on the image in which the arbitrary target inside the body is reflected by the light 153c may be pre-stored. Thus, by applying the brightness of the lesion LE on the image to the data, the distance from the tip portion 153 to the lesion LE, that is, the first distance D, may be calculated.

In an embodiment, the first distance D may be calculated by using an average. In detail, the first distance D may be calculated by averaging distances obtained by applying brightnesses at a plurality of points within the lesion LE, to the data.

In another embodiment, referring to FIG. 9, the calculating of the first distance (S130) may include obtaining the first image i1 when the tip portion 153 is at the first angle a, and the second image i2 when the tip portion 153 is at the second angle b (S132a), and measuring the difference $\Delta\theta$ between the first angle a and the second angle b and the visual disparity $\Delta L$ between the first image i1 and the second image i2 (S132b).

The first angle a and the second angle b are two different arbitrary angles for calculating the first distance D, and may refer to angles between the tip portion 153 and the insertion unit 151 formed by the bending portion 152. Meanwhile, as the angle of the tip portion 153 changes, the position of the image sensor 153a may change, and as the position of the image sensor 153a changes, images may appear as if the position of an arbitrary point W changes. The visual disparity may refer to the degree to which the images appear as if the position of the arbitrary point W changes as the position of the image sensor 153a changes. In detail, the first distance D may be calculated by using [Equation 1] above.

In another embodiment, the first distance D may be calculated by using a stereo method based on a plurality of image sensors 153a.

In another embodiment, light of a particular pattern, such as stripes or grids, may be emitted as structured light toward the lesion LE, and the first distance D may be calculated by measuring the distortion of the pattern of the light returned from the lesion LE to the image sensor 153a.

In another embodiment, the first distance D may be calculated by measuring the travel time of light reflected from the lesion LE after emitting light to the lesion LE.

The endoscopic device 100 may determine the size information R of the lesion based on the first distance D (S140). The size information may be determine based on the lesion size information S on the image and the first distance D.

In an embodiment, the endoscopic device 100 may determine the size information R of the lesion by using a regression model and a proportional value of a size that the size of one pixel on the image actually corresponds to at a point away from the tip portion 153 by the first distance D. Referring to FIGS. 3 and 5, the size information R of the lesion may be obtained by multiplying the proportional value by the number of pixels corresponding to the size information on the image of the bounding box B surrounding the lesion LE. As a specific example, the actual horizontal size RW of the lesion may be calculated by multiplying the horizontal size xw of the bounding box B by the proportional value. The actual vertical size RH of the lesion may be calculated by multiplying the vertical size yh of the bounding box B by the proportional value.

In another embodiment, the endoscopic device 100 may obtain the size information R of the lesion by using the first distance D and the focal length f of the lens 153b. The focal length f of the lens 153b, the sizes xw and yh of the bounding box B on the image, the first distance D, and the actual sizes RW and RH of the lesion may be expressed by an proportional expression as shown in [Equation 2] above.

Thus, the actual sizes RW and RH of the lesion included in the size information R of the lesion may be calculated by using [Equation 3] above, which is a modification of [Equation 2].

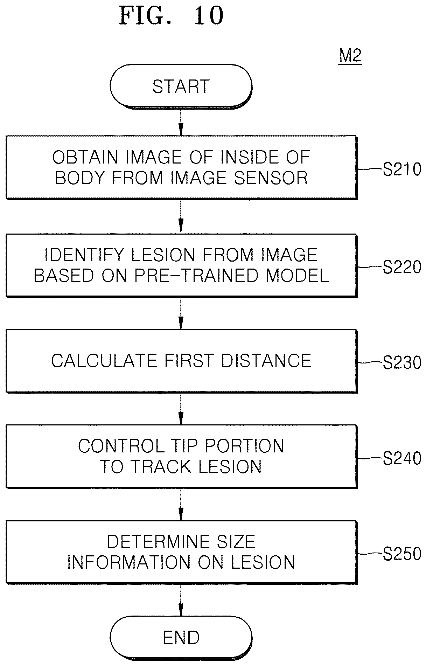

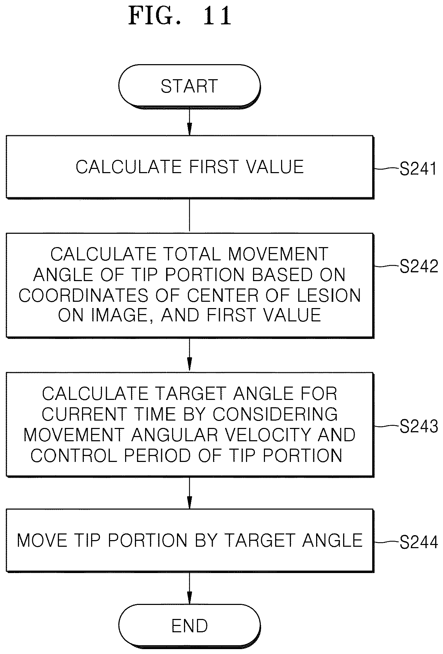

FIG. 10 is a flowchart of a method M2 of obtaining size information on a lesion, according to another embodiment. FIG. 11 is a flowchart showing sub-operations of the controlling of the tip portion 153 (S240), according to an embodiment.

Referring to FIG. 10, the method M2 of obtaining size information on a lesion may include obtaining an image A inside the body from the image sensor 153a (S210), identifying at least one lesion LE from the image A based on the pre-trained model 200 (S220), calculating a first distance D (S230), controlling the tip portion 153 to track the lesion LE (S240), and determining size information on the lesion (S250). Among the operations, the obtaining of the image (S210), the identifying of the lesion (S220), the calculating of the first distance (S230), and the determining of the size information on the lesion (S250) are the same as or similar to those described above regarding the method M1 of obtaining size information on a lesion, and thus, detailed descriptions thereof will be omitted and the differences therebetween will be mainly described.

The endoscopic device 100 may control the tip portion 153 to track the lesion LE (S240). The controlling of the tip portion (S240) may include controlling the tip portion 153 such that the lesion LE is located within the first area.

In an embodiment, the coordinates of the center of the bounding box B may be calculated from the coordinate information on the image for the four line segments of the bounding box B. Referring to FIG. 3, coordinates $(x_o, y_o)$ of the center of the bounding box B of the lesion LE identified by the pre-trained model 200 in the image 300 inside the body output to the display unit 110 may be calculated as in [Equation 4] above from the coordinate information on the image for the four line segments of the bounding box B. The coordinates $(x_o, y_o)$ of the center of the bounding box B may be referred to as the coordinates of the center of the identified lesion LE.

Meanwhile, the angle of view of the image sensor 153a may include the first area. The first area may include the center of the angle of view. Referring to FIG. 6, the portion 310 corresponding to the first area in the image 300' inside the body may include the center of the image 300'. Thus, when a target is located in the first area, the target may be located close to the center of the image output through the display unit 110.

That is, as the tip portion 153 is controlled such that the identified lesion LE is located within the first area, the identified lesion LE may be located close to the center of the image output through the display unit 110. As a result, the identified lesion LE may be continuously output on the display unit 110.

Referring to FIG. 11, the controlling of the tip portion (S240) may include calculating the first value K (S241), calculating the total movement angle TMA of the tip portion based on the coordinates $(x_o, y_o)$ of the center of the lesion on the image, and the first value K (S242), calculating the target angle ag for the current time by considering the movement angular velocity as and the control period cp of the tip portion 153 (S243), and moving the tip portion 153 by the target angle ag for the current time (S244).

The endoscopic device 100 may calculate the first value K (S241). The first value K is the ratio of the difference in position on the image to the change in angle of the tip portion 153. The first value K may be a value necessary to calculate the total movement angle TMA of the tip portion 153.

In an embodiment, the calculating of the first value (S241) may include obtaining the third image i3 when the tip portion 153 is at the third angle c, and the fourth image i4 when the tip portion 153 is at the fourth angle d, and measuring the difference $\Delta\delta$ between the third angle c and the fourth angle d, and the position difference $\Delta X$ between the third image i3 and the fourth image i4.

The third angle c and the fourth angle d are two different arbitrary angles for calculating the first value K, and may refer to angles between the tip portion 153 and the insertion unit 151 formed by the bending portion 152. Meanwhile, the position difference $\Delta X$ between the third image i3 and the fourth image i4 may include a difference in coordinates on images for an arbitrary point X inside the body. That is, it may include the difference between the coordinates of the arbitrary point X on the third image i3 and the coordinates of the arbitrary point X on the fourth image i4.

Referring to FIG. 7, the third image i3 may be obtained with the image sensor 153a when the tip portion 153 is caused to be at the third angle c by manipulating the bending portion 152. Here, the coordinates of the arbitrary point X inside the body on the image may be $(x_c, y_c)$. The method of designating the arbitrary point X is not particularly limited. In an embodiment, the arbitrary point X may be the position of a blood vessel having a particular shape inside the body found on the image.

Again, the fourth image i4 may be obtained with the image sensor 153a when the tip portion 153 is caused to be at the fourth angle d by manipulating the bending portion 152. Here, the coordinates of the arbitrary point X inside the body on the image may be $(x_d, y_d)$.

The coordinate difference between the third image i3 and the fourth image i4 may be expressed as $(\Delta x, \Delta y)$, which is the difference between the coordinates of the arbitrary point X on the images. Here, $\Delta x = x_d - x_c$, and $\Delta y = y_d - y_c$. By using this, the position difference $\Delta X$ between the third image i3 and the fourth image i4 is expressed as [Equation 5] above.

By applying the difference $\Delta\delta$ between the third angle c and the fourth angle d and the position difference $\Delta X$ between the third image i3 and the fourth image i4 to [Equation 6] above, the first value K may be calculated.

In an embodiment, the calculating of the first value (S241) may use the first image i1 and the second image i2 obtained in the calculating of the first distance (S130). In detail, as the difference $\Delta\delta$ between the third angle c and the fourth angle d, the difference $\Delta\theta$ between the first angle a and the second angle b may be used. In addition, as the position difference $\Delta X$, the difference between coordinates (xa, ya) of the arbitrary point W on the first image i1 and coordinates (xb, yb) of the arbitrary point W on the second image i2 may be used.

The endoscopic device 100 may calculate the total movement angle TMA of the tip portion based on the coordinates (xo, yo) of the lesion on the image, and the first value K (S242). The total movement angle TMA refers to a total angle that the tip portion 153 needs to move to move the center coordinates (xo, yo) of the lesion LE to the center coordinates (0, 0) on the image. The length Q from the center of the lesion LE to the center of the image is as shown in [Equation 7] above.

The total movement angle TMA may be calculated by applying the length Q from the center of the lesion LE to the center of the image, and the calculated first value K to [Equation 8] above.

The endoscopic device 100 may calculate the target angle ag for the current time by considering the movement angular velocity as and the control period cp of the tip portion 153 (S243). The movement angular velocity as and the control period cp of the tip portion 153 may be set in advance.

In an embodiment, the target angle ag for the current time may be calculated through a polynomial trajectory. As a specific example, the tip portion 153 may be set to move by the total movement angle TMA at a constant velocity. The preset movement angular velocity as of the tip portion 153 may be 30°/s, and the preset control period cp may be 2 ms. In this case, the target angle ag for the current time may be 30°/s $\times$ 2ms = 0.2 °. However, this is only an example for description and the disclosure is not limited thereto.

In another embodiment, the target angle ag for the current time may be calculated through a Bezier curve trajectory. That is, the method of calculating the target angle ag for the current time is not limited to the polynomial trajectory. In other words, the target angle ag for the current time may be calculated through various trajectories that may occur to one of skill in the art.

The endoscopic device 100 may move the tip portion 153 by the target angle ag for the current time (S244). The endoscopic device 100 may calculate a force required to move the tip portion 153 by the target angle ag for the current time, by considering the dynamic characteristics of the bending portion 152. The endoscopic device 100 may deliver information about the calculated force to the driving unit 130 to move the tip portion 153 by the target angle ag for the current time.

The identifying of the lesion (S220), the calculating of the first distance (S230), and the controlling of the tip portion (S240) may be repeatedly performed until the lesion LE is located in the first area. Accordingly, the tip portion 153 may track the lesion LE, and the lesion LE may be continuously output on the display unit 110.

As the controlling of the tip portion (S240) is further included, the lesion LE is located close to the center of the field of view C of the image sensor 153a, and thus, the convenience and accuracy of calculating the first distance D may be improved. Accordingly, the reliability of the size information R of the lesion may be improved. In addition, even a low-skilled user is able to track and observe the lesion LE without difficulty, and the quality of treatment may be improved as the user is able to focus on observation, treatment, and procedures of the identified lesion LE without having to pay attention to manipulation of the scope.

The disclosure is described above with reference to the embodiments illustrated in the drawings, but the embodiments are only examples. Those of skill in the art will understand that various modifications and other equivalent embodiments may be derived from the embodiments. Therefore, the true technical protection scope of the disclosure should be determined by the appended claims.

The particular technological details described in the embodiments are illustrative examples, and are not intended to limit the technological scope of the embodiments. In order to briefly and clearly describe the disclosure, descriptions of general techniques and configurations in the art may be omitted. Furthermore, line connections or connection members between elements depicted in the drawings represent functional connections and/or physical or circuit connections by way of example, and in actual applications, they may be replaced or embodied with various suitable additional functional connections, physical connections, or circuit connections. In addition, no item or component is essential to the practice of the disclosure unless the item or component is specifically described as being "essential" or "critical".

As used herein, the term "the" and other demonstratives similar thereto may include a singular form and plural forms, unless specifically limited. Furthermore, recitation of ranges of values herein are merely intended to serve as a shorthand method of referring individually to each separate value falling within the range, unless otherwise indicated herein, and each separate value is incorporated into the specification as if it were individually recited herein. In addition, the operations of all methods described herein may be performed in any suitable order unless otherwise indicated herein or otherwise clearly contradicted by context. The embodiments are not limited to the described order of the operations. The use of any and all examples, or exemplary language (e.g., "and the like") provided herein, is intended merely to better illuminate embodiments and does not pose a limitation on the scope of the embodiments unless otherwise claimed. In addition, those of skill in the art will understand that various modifications, combinations, and adaptations may be configured according to design conditions and factors without departing from the following claims and equivalents thereof.

[Explanation of Reference Numerals]

| | | | |
|---|---|---|---|
| 1: | Endoscopic device | | |
| 110: | Display unit | 120: | Control unit |
| 130: | Driving unit | 140: | Manipulation unit |
| 150: | Scope | 151: | Insertion unit |
| 152: | Bending portion | 153: | Tip portion |
| 153a: | Image sensor | 153b: | Lens |
| 153c: | Light | | |
| 200: | Model | | |
| M1, M2: | Method of obtaining size information on a lesion | | |
| LE: | Lesion | | |
| B: | Bounding box | | |
| D: | First distance | | |
| S: | Lesion size information on an image | | |
| R: | Size information on a lesion | | |
| K: | First value | | |

**Claims**

1. A method of obtaining size information on a lesion, the method comprising:

   obtaining an image of inside of a body from an image sensor;
   identifying at least one lesion from the image based on a pre-trained model; and
   determining size information on the lesion.

2. The method of claim 1, further comprising calculating a first distance,
   wherein the determining of the size information on the lesion comprises determining the size information on the lesion based on the first distance.

3. The method of claim 2, wherein the calculating of the first distance comprises calculating the first distance based on brightness information on the lesion expressed in the image.

4. The method of claim 2, wherein the calculating of the first distance comprises:

   obtaining a first image when a tip portion is at a first angle, and a second image when the tip portion is at a second angle; and
   measuring a difference between the first angle and the second angle, and a visual disparity between the first image and the second image.

5. The method of claim 1, wherein the identifying of the lesion comprises determining lesion size information on the image, which indicates size information on the lesion on the image.

6. The method of claim 1, further comprising controlling the tip portion to track the lesion.

7. An endoscopic device comprising:

   a tip portion comprising an image sensor; and
   a control unit configured to obtain an image of inside of a body from the image sensor, identify at least one lesion from the image based on a pre-trained model, and determine size information on the lesion.

8. The endoscopic device of claim 7, wherein the control unit is further configured to calculate a first distance, and determine the size information on the lesion based on the first distance.

9. The endoscopic device of claim 8, wherein the control unit is further configured to calculate the first distance based on brightness information on the lesion expressed in the image.

**10.** The endoscopic device of claim 8, wherein the control unit is further configured to obtain a first image when the tip portion is at a first angle, and a second image when the tip portion is at a second angle, and measure a difference between the first angle and the second angle, and a visual disparity between the first image and the second image.

**11.** The endoscopic device of claim 7, wherein the control unit is further configured to determine lesion size information on the image, which indicates size information on the lesion on the image.

**12.** The endoscopic device of claim 11, wherein the lesion size information on the image comprises a bounding box displayed to surround the lesion.

**13.** The endoscopic device of claim 7, wherein the control unit is further configured to control the tip portion to track the lesion.

**14.** The endoscopic device of claim 13, wherein an angle of view of the image sensor comprises a first area, and the control unit is further configured to control the tip portion such that the lesion is located within the first area.

**15.** The endoscopic device of claim 13, wherein the control unit is further configured to calculate a first value.

# FIG. 1

100

FIG. 2

# FIG. 3

# FIG. 4

# FIG. 5

FIG. 6

EP 4 607 471 A1

FIG. 7

i1

(xc,yc)
X

i2

(xd,yd)
X

(Δx,Δy)

21

# FIG. 8

M1

START

OBTAIN IMAGE OF INSIDE OF
BODY FROM IMAGE SENSOR — S110

IDENTIFY LESION FROM IMAGE
BASED ON PRE-TRAINED MODEL — S120

CALCULATE FIRST DISTANCE — S130

DETERMINE SIZE INFORMATION ON LESION — S140

END

# FIG. 9

S130

CALCULATE
FIRST DISTANCE

CALCULATE FIRST DISTANCE
BASED ON BRIGHTNESS
INFORMATION ON LESION
— S131

OR

OBTAIN FIRST IMAGE
AND SECOND IMAGE
— S132a

MEASURE ANGLE DIFFERENCE
AND VISUAL DISPARITY
BETWEEN IMAGES
— S132b

# FIG. 10

<u>M2</u>

START

OBTAIN IMAGE OF INSIDE OF
BODY FROM IMAGE SENSOR ———S210

IDENTIFY LESION FROM IMAGE
BASED ON PRE-TRAINED MODEL ———S220

CALCULATE FIRST DISTANCE ———S230

CONTROL TIP PORTION
TO TRACK LESION ———S240

DETERMINE SIZE
INFORMATION ON LESION ———S250

END

# FIG. 11

```
         ┌─────────┐
         │  START  │
         └────┬────┘
              ▼
┌────────────────────────────────┐
│     CALCULATE FIRST VALUE      │─── S241
└────────────────┬───────────────┘
                 ▼
┌────────────────────────────────┐
│   CALCULATE TOTAL MOVEMENT     │
│  ANGLE OF TIP PORTION BASED ON │─── S242
│ COORDINATES OF CENTER OF LESION│
│     ON IMAGE, AND FIRST VALUE  │
└────────────────┬───────────────┘
                 ▼
┌────────────────────────────────┐
│   CALCULATE TARGET ANGLE FOR   │
│    CURRENT TIME BY CONSIDERING │─── S243
│  MOVEMENT ANGULAR VELOCITY AND │
│  CONTROL PERIOD OF TIP PORTION │
└────────────────┬───────────────┘
                 ▼
┌────────────────────────────────┐
│ MOVE TIP PORTION BY TARGET ANGLE│─── S244
└────────────────┬───────────────┘
                 ▼
           ┌─────────┐
           │   END   │
           └─────────┘
```

Europäisches
Patentamt

European
Patent Office

Office européen
des brevets

## EUROPEAN SEARCH REPORT

**Application Number**

EP 24 15 9273

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2024/049942 A1 (YOSHIOKA MASATO [JP] ET AL) 15 February 2024 (2024-02-15) | 1-3,5-9, 11-15 | INV.<br>G06V10/25 |
| Y | * paragraphs [0002], [0034], [0038], [0039], [0057], [0058], [0060], [0061] * | 4,10 | G06V10/82<br>A61B1/00<br>A61B5/107<br>G06T7/00 |
| | - - - - - | | |
| Y | US 6 459 481 B1 (SCHAACK DAVID F [US]) 1 October 2002 (2002-10-01) * column 11, line 29 - column 13, line 50; figures 1-6 * | 4,10 | |
| | - - - - - | | |

**TECHNICAL FIELDS SEARCHED (IPC)**

G06V
A61B
G06T

The present search report has been drawn up for all claims

| Place of search | Date of completion of the search | Examiner |
|---|---|---|
| The Hague | 11 June 2024 | Natanni, Francesco |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 24 15 9273

This annex lists the patent family members relating to the patent documents cited in  the above-mentioned European search report.
The members are as contained in the European Patent Office EDP file on
The European Patent Office is in no way liable for these particulars which are merely given for the purpose of information.

11-06-2024

| Patent document cited in search report | Publication date | Patent family member(s) | Publication date |
|---|---|---|---|
| US 2024049942 A1 | 15-02-2024 | JP WO2022224859 A1 | 27-10-2022 |
| | | US 2024049942 A1 | 15-02-2024 |
| | | WO 2022224859 A1 | 27-10-2022 |
| US 6459481 B1 | 01-10-2002 | NONE | |

EPO FORM P0459

For more details about this annex : see Official Journal of the European Patent Office, No. 12/82